(19)

Europäisches
Patentamt
European
Patent Office
Office européen
des brevets

(11) **EP 3 686 596 A2**

(12) **EUROPEAN PATENT APPLICATION**

(43) Date of publication:
    **29.07.2020 Bulletin 2020/31**

(51) Int Cl.:
    ***G01N 33/00*** (2006.01)    ***G16B 40/00*** (2019.01)

(21) Application number: **20151355.3**

(22) Date of filing: **13.01.2020**

(84) Designated Contracting States:
    **AL AT BE BG CH CY CZ DE DK EE ES FI FR GB
    GR HR HU IE IS IT LI LT LU LV MC MK MT NL NO
    PL PT RO RS SE SI SK SM TR**
    Designated Extension States:
    **BA ME**
    Designated Validation States:
    **KH MA MD TN**

(30) Priority: **28.01.2019 SG 10201900755W**

(71) Applicant: **Wilmar International Limited
    Singapore 088830 (SG)**

(72) Inventor: **Lim Jungliang, Kevin
    544815 Singapore (SG)**

(74) Representative: **Goddar, Heinz J.
    Boehmert & Boehmert
    Anwaltspartnerschaft mbB
    Pettenkoferstrasse 22
    80336 München (DE)**

(54) **METHODS AND SYSTEM FOR PROCESSING LIPID CONTENTS OF AT LEAST ONE OIL SAMPLE, SIMULATING AT LEAST ONE TRAINING SAMPLE, AND FOR PREDICTING A BLENDING FORMULA**

(57)    According to embodiments, a method for processing lipid contents of at least one oil sample and simulating at least one training sample is provided. The method includes receiving a first dataset of values associated with lipid contents of the at least one oil sample; applying a multivariate mixture model to the first dataset of values to simulate an intermediate dataset; and simulating the at least one training sample based on at least part of the simulated intermediate dataset. According to further embodiments, a method for predicting the blending formula is also provided. The prediction method includes receiving a dataset of values associated with lipid contents of a blended oil sample; and processing the dataset using a single prediction model capable of generating a prediction of at least two constituent oils in the blended oil sample. A system therefor is also described.

FIG. 1A

## Description

### Technical Field

Various embodiments relate to a method for processing lipid contents of at least one oil sample and simulating at least one training sample, a method for predicting the blending formula, a method for identifying a blind oil sample, and an apparatus or system therefor.

### Background

Quantitative prediction of blended oil ratios based on lipid content, such as fatty acid profile, is a difficult task. A difficulty is in modeling the space of possible blend formulas. Existing methods in the art focus on a small number of possible constituent oils, whereby the space is substantially reduced. For a blended formula of more than three oils, the combinations of possible constituent oils grow exponentially.

To add on to this difficulty, quantitative modeling requires a number of training examples per set of constituent oil formulations. A further difficulty is that conventional modeling techniques such as partial least squares (PLS1/PLS2) and linear regression (MLR) are limited by the size of a dataset of information. The large number of samples required for modeling (as mentioned above) makes this task computationally expensive and nearly impossible to solve.

An upscaling difficulty is that PLS1/PLS2 and MLR depend on an assumption that a linear relationship may be observed between the features (fatty acid contents) and blended formula. Existing methods may show that in the space of blended oil with small number of constituent oils, this linear relationship may hold true. However, for more complex blends involving more than three oils, this assumption may be invalid.

Prediction of blended oil formula has been published. For example, applications in predicting mustard, rapeseed and soybean oil blends have been explored. A ternary blend system is far less complex compared to real-life industrial scenarios where as many as nine oil types may be used for blend formulation.

For example, in one publication, random picking of constituent oil and mixing proportions picked from a random uniform distribution normalized to unity were disclosed. This methodology is simple and direct. However, if the sample size of constituent oil is significantly large, random picking may not consider the entire population of pure constituent oils. Further, the resulting mixing proportions may not be symmetric around the constituent oils. Such effects may not be significantly pronounced when smaller number of constituents is considered. However, these effects cannot be ignored when large number of constituent oils is involved (e.g. up to nine constituent oil blends).

In another publication, simulation of constituent oils using PERT distribution and exhaustive enumeration of mixing proportions were described. Although a sample of the population of each constituent oil is explored, which elevates from sampling bias, as described above, the PERT distribution adopts a univariate view. In that, each fatty acid is generated independently and does not affect other fatty acid values. This is not representative of a real-life scenario where multivariate variances between fatty acid values need to be considered. The approach of using exhaustive enumeration of mixing proportions is relatively limited as it may be plausible for only systems with small number of constituent oils (e.g. ternary).

Existing approaches infer the linear relationship between the blended oil FAC (fatty acid contents) and the blend formula (mix proportions) by partial least squares (PLS) or canonical discriminant analysis (CDA). Common to these techniques, a matrix decomposition of multivariate features and multivariate responses are performed in such a way that correlation to target responses is maximized.

Matrix decomposition works only for small number of constituent oils (e.g. using not more than three oils in a ternary system). As previously mentioned, for small number of constituent oils that are obviously different in a reduced dimension space, a linear relationship may be tested. If a larger number of constituent oils were to be considered, then there may be increased chances that this linear relationship may not hold. In addition, algorithms based on matrix decomposition do not scale well to increased number of constituent oils due to the combinatorial increase in possible mixing ratios. To possibly reduce the complexity, another publication has suggested formulating the problem into a classification problem (qualitative) instead, i.e. merely identifying blended or not, or adulterated or not.

Thus, there is a need for methods and system of processing lipid contents of an oil sample and simulating a training sample, and for quantitatively predicting the blending formula, thereby addressing at least the problems mentioned hereinabove and providing for specific industry needs working on complex blends and blind samples.

### Summary

According to an embodiment, a method for processing lipid contents of at least one oil sample and simulating at least one training sample is provided. The method may include receiving a first dataset of values associated with lipid contents of the at least one oil sample, wherein the lipid content of the at least one oil sample includes a first lipid content and a second lipid content; applying a multivariate mixture model to the first dataset of values to simulate an intermediate dataset, wherein the multivariate mixture model is capable of accounting for at least a multivariate variance between the value associated with the first lipid content and the value associated with the second lipid content; and simulating the at least one training sample based on at least part of the simulated intermediate dataset, wherein the at least one simulated training sample is represented by a second dataset of values associated with lipid contents of the at least one training sample.

According to an embodiment, a method for predicting a blending formula of a blended oil sample is provided. The method includes receiving a dataset of values associated with lipid contents of the blended oil sample; providing a single prediction model capable of generating a prediction of at least two constituent oils in the blended oil sample; and processing the at least part of the dataset using the single prediction model, wherein the single prediction model is trained by a training dataset, wherein the training dataset comprises a second dataset of values associated with lipid contents of the at least one training sample simulated by a method in accordance with various embodiments.

According to an embodiment, a method for identifying a blind oil sample is provided. The method includes receiving a dataset of values associated with lipid contents of the blind oil sample; and applying a multivariate mixture model comprising groups of lipid parameters to the dataset of values to identify the blind oil sample, wherein the groups of lipid parameters is learned by the multivariate mixture model based on lipid contents of at least one preceding oil sample, wherein the lipid contents of the at least one preceding oil sample include a first lipid content and a second lipid content, and wherein the multivariate mixture model is capable of accounting for at least a multivariate variance between a value associated with the first lipid content and a value associated with the second lipid content.

According to an embodiment, a method for processing lipid contents of at least one oil sample to simulate at least one blend sample and determining compliance of the at least one blend sample with a prescribed requirement is provided. The method includes receiving a first dataset of values associated with lipid contents of the at least one oil sample; simulating the at least one blend sample based on at least part of the first dataset, wherein the at least one simulated blend sample is represented by a second dataset of values associated with lipid contents of the at least one simulated blend sample; and determining whether each value of the second dataset for each simulated blend sample falls within a pre-determined range of a corresponding lipid content in accordance with the prescribed requirement such that if it is determined affirmative, the simulated blend sample complies with the prescribed requirement, and if it is determined negative, the simulated blend sample fails compliance with the prescribed requirement.

According to an embodiment, a computer readable storage medium including computer readable instructions operable when executed by a computer to process lipid contents of at least one oil sample and simulate at least one training sample; and/or to predict a blending formula of a blended oil sample; and/or to identify a blind sample; and/or to process lipid contents of at least one oil sample to simulate at least one blend sample and determine compliance of the at least one blend sample with a prescribed requirement is provided. The computer readable instructions may be configured to perform the methods according to various embodiments.

According to an embodiment, an apparatus or system is provided. The apparatus or system may include a receiving unit configured to receive a dataset of values associated with lipid contents of at least one oil sample or a blended oil sample, or a blind oil sample; a memory for at least storing a multivariate mixture model capable of accounting for at least a multivariate variance between the lipid contents of the at least one oil sample, a single prediction model capable of generating a prediction of a blending formula of the blended oil sample, and a probability distribution model capable of simulating a blend sample based on at least part of the dataset; and a processor configured to access the multivariate mixture model to perform steps of the method according to various embodiments for processing the lipid contents of the at least one oil sample and simulating at least one training sample, or to perform steps of the method according to various embodiments for identifying the blind oil sample; the single prediction model stored in the memory to perform steps of the method according to various embodiments for generating a prediction of the blending formula of the blended oil sample; and the probability distribution model stored in the memory to perform steps of the method according to various embodiments for processing lipid contents of the at least one oil sample to simulate at least one blend sample and determining compliance of the at least one blend sample with a prescribed requirement.

### Brief Description of the Drawings

In the drawings, like reference characters generally refer to like parts throughout the different views. The drawings are not necessarily to scale, emphasis instead generally being placed upon illustrating the principles of the invention. In the following description, various embodiments of the invention are described with reference to the following drawings, in which:

FIG. 1A shows a flow chart illustrating a method for processing lipid contents of at least one oil sample and simulating at least one training sample, according to various embodiments.
FIG. 1B shows a flow chart illustrating a method for predicting a blending formula of a blended oil sample, according to various embodiments.

FIG. 1C shows a schematic view of an apparatus or system for processing lipid contents of at least one oil sample and simulating at least one training sample; and for predicting a blending formula of a blended oil sample, according to various embodiments.

FIG. 2A shows graphical representations illustrating various exemplary configurations of the Gaussian mixture model having constraint on the shape parameters (taken from Scurucca et al, "mclust 5 : Clustering, Classification and Density Estimation Using Gaussian Finite Mixture Models", The R Journal Vol.8/1, August 2016).

FIG. 2B shows a geometrical representation of a simplified hypothetical example involving two pure oil samples, each of which having two fatty acid contents as variables.

FIG. 2C shows a graphical representation illustrating the clusters of 9 original oil types in a latent space based on fatty acid contents.

FIG. 2D shows a graphical representation illustrating the clusters of 12 groups of oils identified by the Gaussian mixture model based on the fatty acid contents (FAC).

FIG. 2E shows a graphical representation of an example clustering 5 original oil types (original label) based on triacylglyceride (TAG) contents.

FIG. 2F shows a graphical representation illustrating the clusters of 5 groups of oil identified by the Gaussian mixture model based on triacylglyceride (TAG) contents.

FIG. 2G shows a graphical representation illustrating real and simulated data of pure oils from the Gaussian mixture model in a linear-transformed space, in accordance with one embodiment.

FIG. 2H shows a graphical representation illustrating real and simulated data of pure oils from the Gaussian mixture model in a non-linear transformed space, in accordance with one embodiment.

FIG. 3A shows a simplified graphical representation using three oil types (A, B, C) for blending and illustrating drawing from a random uniform distribution normalized to unity, as provided by existing approaches.

FIG. 3B shows a simplified graphical representation using the same three oil types (A, B, C) as in FIG. 3A for blending and illustrating from the Dirichlet distribution, in accordance with one embodiment.

FIG. 4A shows a box plot drawing 9 mixing proportions normalized from a uniform distribution, as provided by existing approaches.

FIG. 4B shows a box plot drawing 9 mixing proportions from the Dirichlet distribution, in accordance with one embodiment.

FIG. 5 shows a schematic representation of the deep learning model, in accordance with various embodiments.

FIG. 6A shows a graph representing the final PLS results for 9 constituent oils.

FIG. 6B shows a graph representing the final deep learning results for the same 9 constituent oils, in accordance with one embodiment.

## Detailed Description

The following detailed description refers to the accompanying drawings that show, by way of illustration, specific details and embodiments in which the invention may be practiced. These embodiments are described in sufficient detail to enable those skilled in the art to practice the invention. Other embodiments may be utilized and structural, logical, and electrical changes may be made without departing from the scope of the invention. The various embodiments are not necessarily mutually exclusive, as some embodiments can be combined with one or more other embodiments to form new embodiments.

Embodiments described in the context of one of the methods or devices/apparatus are analogously valid for the other methods or devices/apparatus. Similarly, embodiments described in the context of a method are analogously valid for a device, and vice versa.

Features that are described in the context of an embodiment may correspondingly be applicable to the same or similar features in the other embodiments. Features that are described in the context of an embodiment may correspondingly be applicable to the other embodiments, even if not explicitly described in these other embodiments. Furthermore, additions and/or combinations and/or alternatives as described for a feature in the context of an embodiment may correspondingly be applicable to the same or similar feature in the other embodiments.

In the context of various embodiments, the articles "a", "an" and "the" as used with regard to a feature or element include a reference to one or more of the features or elements.

In the context of various embodiments, the phrase "substantially" may include "exactly" and a reasonable variance.

In the context of various embodiments, the term "about" or "approximately" as applied to a numeric value encompasses the exact value and a reasonable variance.

As used herein, the term "and/or" includes any and all combinations of one or more of the associated listed items.

As used herein, the phrase of the form of "at least one of A or B" may include A or B or both A and B.

Correspondingly, the phrase of the form of "at least one of A or B or C", or including further listed items, may include any and all combinations of one or more of the associated listed items.

Various embodiments may provide simulated blended oil fatty acid contents (FAC) for modeling and prediction of blended oil constituents and blending formulas.

Various embodiments may provide an application of integrating multiple machine learning techniques (including Gaussian mixture models and deep learning) to construct a model capable of predicting oil blends of up to nine oil types.

Various embodiments may provide for in-silico blended oil FAC simulation and detection of blended oil formulation for large number of constituent oils (e.g. for more than three constituent oils).

The in-silico blended oil FAC simulation may be considered as a weighted sum of constituent oil proportions, and generally includes two steps of (i) determining the choice of constituent oils and (ii) determining the mixing proportions.

Various embodiments may provide Monte Carlo simulation of constituent oils using parameters learned from a Gaussian Mixture Model and mixing proportions drawn from a Dirichlet distribution. This approach considers a multivariate view.

In relation to the detection of blended oil formulation, deep learning may be used to resolve or at least address the issue inferred by the linear relationship between the blended oil FAC and the blend formula. For example, deep learning is able to model non-linear relationships. In other words, deep learning models may handle non-linear structures in data significantly well.

Further, instead of matrix decomposition, deep learning is based on forward and backward propagation. As deep learning is trained on batches of data, this approach is able to scale well with the combinatorial increase in training examples.

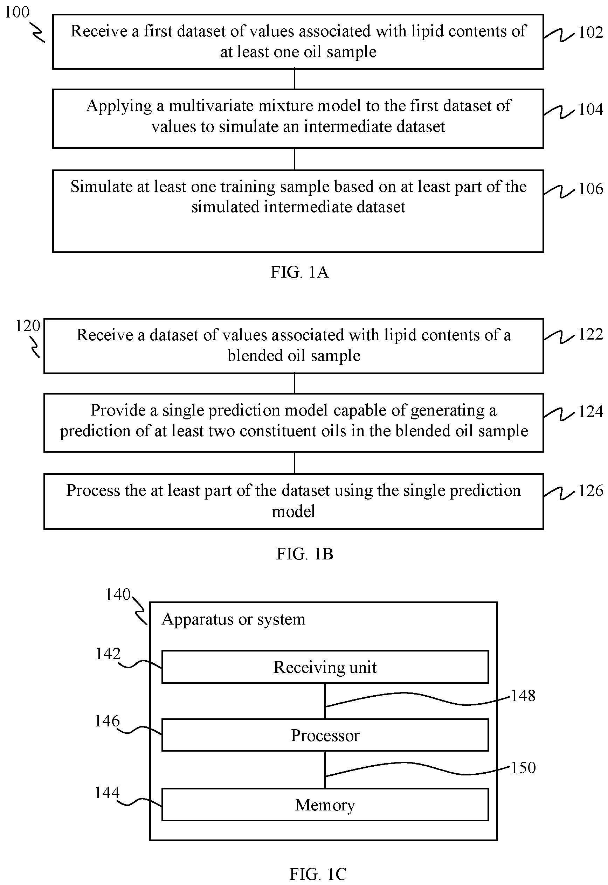

FIG. 1A shows a flow chart illustrating a method for processing lipid contents of at least one oil sample and simulating at least one training sample 100, according to various embodiments. In FIG. 1A, at Step 102, a first dataset of values associated with lipid contents of the at least one oil sample is received. The lipid contents of the oil sample may include a first lipid content and a second lipid content. At Step 104, a multivariate mixture model is applied to the first dataset of values to simulate an intermediate dataset. The multivariate mixture model is capable of accounting for at least a multivariate variance between the value associated with the first lipid content and the value associated with the second lipid content. The terms "first" and "second" in relation to the first lipid content and the second lipid content merely refers to one lipid content different from another lipid content. In this context, the terms "first" and "second" do not refer to elements in any particular order in a list. For example, the lipid content may include the fatty acid content of C8:0, C10:1, C12:0, C12:1, C14:2, C14:1, C16:1, C18:1, C18:2, C18:3, C20:4, C20:5, C22:1, C22:6, and so on. Thus, if the first lipid content refers to the fatty acid content of C16:1, then the second lipid content may refer to the fatty acid content of any carbon bond other than C16:1. At Step 106, at least one training sample is simulated based on at least part of the simulated intermediate dataset. The at least one simulated training sample is represented by a second dataset of values associated with lipid contents of the at least one simulated training sample.

In the context of various embodiments, the term "processed" or "processing" may mean analyzing, or mathematically or computationally evaluating.

The processing of the lipid contents includes the processing of data including or containing the lipid contents.

The method 100 may be a method of processing the lipid contents of the at least one oil sample and simulating the at least one training sample for training a prediction model for predicting a blending formula, for example, at least two constituent oils in a blended oil sample.

In various embodiments, the multivariate mixture model may include a Gaussian mixture model or a multivariate Gaussian mixture model. The multivariate mixture model may generate a probability distribution (sub-population) with a multivariate view. For example, the multivariate mixture model may be constructed and constrained by a geometric characteristic. The geometric characteristic may include a diagonal or ellipsoidal distribution with equal volume and equal shape. Other geometric characteristics may include a diagonal or ellipsoidal distribution with unequal volume and equal shape; or unequal volume and unequal shape; or equal volume and unequal shape.

In various embodiments, the step of applying the multivariate mixture model to the first dataset of values at 104 may include learning groups of lipid parameters from the first dataset of values; and simulating, based on the groups of lipid parameters, the intermediate dataset.

In other words, each group of lipid parameters may be learned or generated by the multivariate mixture model when the multivariate mixture model is used to analyze lipid contents of the at least one oil sample, which may be a known sample. For example, the at least one oil sample may include a pure oil sample. In other examples, the at least one oil sample may also include an oil blend.

Each group of lipid parameters may include a vector of average (mean) values representing likely lipid percentages of the at least one oil sample and a covariance matrix representing a spread within and across the likely lipid percentages of the at least one oil sample.

In various embodiments, the step of simulating the at least one training sample 106 may include performing a Monte Carlo simulation. The simulation may be used to generate draws from a probability distribution, thereby resulting in the type(s) of constituent oil(s).

In other words, the step of simulating the at least one training sample 106 may include selecting from the at least part of the simulated intermediate dataset and drawing a mixing proportion from a probability distribution to obtain the at least one simulated training sample. For example, the probability distribution may include a Dirichlet distribution.

In various embodiments, the Dirichlet distribution may include symmetric or asymmetric properties. In one embodiment, the use of the Dirichlet distribution eliminates sampling bias since the Dirichlet distribution is able to sample in a way that is symmetric across the constituent oils.

The number of dimensions of the Dirichlet distribution may be determined by the number of constituent oils from the stimulated intermediate dataset.

For example, the number of dimensions of the Dirichlet distribution may be more than or equal to two.

In various embodiments, the number of dimensions may be 3, 4, or 5, or 6, or 7, or 8, or 9, or more.

It should be appreciated that other probability distributions, for example, a uniform distribution normalized to unity, a normal distribution or any other continuous distribution, may also be applicable with different effects and efficiencies on a training dataset that includes the at least one simulated training sample. In one example, by selecting a mixing proportion determined from a uniform distribution normalized to unity may result in requiring more number of training samples for training a single prediction model to predict a blending formula.

In various embodiments, the lipid contents of the oil sample may include fatty acid contents of the oil sample obtainable using a gas chromatography - flame ionization detector (GC-FID) or photonic detection. For example, the photonic detection may include surface-enhanced Raman spectroscopy (SERS). It should also be appreciated that other lipid contents may be considered, for example, including but not limited to triacylglyceride (TAG) contents.

Various embodiments may provide a method for identifying a blind oil sample. The method may include receiving a dataset of values associated with lipid contents of the blind oil sample; and applying a multivariate mixture model including groups of lipid parameters to the dataset of values to identify the blind oil sample. The groups of lipid parameters may be learned by the multivariate mixture model based on lipid contents of at least one preceding oil sample. The lipid contents of the at least one preceding oil sample may include a first lipid content and a second lipid content, the multivariate mixture model is capable of accounting for at least a multivariate variance between a value associated with the first lipid content and a value associated with the second lipid content. The multivariate mixture model here may be described in similar context with the multivariate mixture model described in Step 104 of FIG. 1A. The at least one preceding oil sample may include a known sample of pure oil or blend. In the context of various embodiments, the method for identifying the blind oil sample may also refer to a method of classifying the blind oil sample.

FIG. 1B shows a flow chart illustrating a method for predicting a blending formula of a blended oil sample 120, according to various embodiments. In FIG. 1B, at Step 122, a dataset of values associated with lipid contents of the blended oil sample is received. At Step 124, a single prediction model capable of generating a prediction of at least two constituent oils in the blended oil sample is provided. At Step 126, the at least part of the dataset is processed using the single prediction model. The single prediction model may be trained by a training dataset, wherein the training dataset may include a second dataset of values associated with lipid contents of the at least one training sample simulated by a method 100 of FIG. 1A. When training the single prediction model, the training dataset may further include a dataset of values associated with lipid contents of known oil blend(s). In other words, the training dataset may involve the lipid contents of known oil blend(s), and the lipid contents of the at least one simulated training sample obtainable from the method 100 of FIG. 1A. In one example, the received dataset of values of Step 122 may comprise at least part of the second dataset of values associated with the lipid contents of the at least one training sample simulated by a method 100 of FIG. 1A. The at least one simulated training sample may include one or more simulated constituent oil, or one or more simulated blended oil, or mixtures thereof. On the other hand, when the trained single prediction model is employed for a blind test, the blended oil sample is a blind sample. The single prediction model may also learn from the results of the blind test for further training.

Various embodiments may provide modeling multiple quantitative constituent oil outcomes in one single model. This advantageously eliminates use of multiple models and human interpretation of intermediate results in existing approaches, e.g. when performing PLS1.

The single prediction model may be a linear model. For example, a quantitative prediction of two variables may be realized using PLS2. Generally, PLS2 may enable prediction of outcomes that generally do not require corroboration and may be more easily interpreted, as compared to PLS1.

In one embodiment, the single prediction model may be a non-linear model. The single prediction model may include a deep neural network (DNN). The DNN may be with multiple layers between an input layer and an output layer. The DNN may be a feedforward network in which data flows from the input layer to the output layer without looping back. The input layer may receive one or multiple inputs. The output layer may provide one or multiple outputs. For example, the deep neural network may be supervised.

The single prediction model may be for modeling complex traits and blends. It is believed that these traits cause the data (lipid contents, e.g., fatty acid contents) to have non-linear structures. Deep learning models are purposed for non-linear pattern discovery, and thus provide a significantly good and useful tool to analyze and assess the non-linearity of the data.

In various embodiments, the single prediction model may be constructed from a matrix of features or trained by the matrix of features. For example, each feature may correspond to each lipid content of the blended oil sample or each lipid content of the training dataset.

In various embodiments, the blended oil sample may include a mixture of pure oils selected from the group consisting of a peanut oil sample, an olive oil sample, a corn oil sample, a coconut oil sample, a cottonseed oil sample, a palm oil sample, a canola oil sample, a safflower oil sample, a sesame oil sample, a soybean oil sample, a sunflower oil sample, a camellia seed oil sample, a linseed (flaxseed) oil sample, and a sample of oil with a relatively higher proportion of certain fatty acids, for example, high erucic acid rapeseed oil, low erucic acid rapeseed oil, or high oleic acid sunflower oil.

In various embodiments, the method 120 may further include the prediction of at least two constituent oils in the blended oil sample. The step of generating the prediction of the least two constituent oils in the blended oil sample may be performed after the step of processing the at least part of the dataset using the single prediction model at Step 126. In other words, the output layer of the single prediction model may provide multiple outputs of the at least two constituent oils or variables. For example, the method 120 may include generating the prediction of nine constituent oils in the blended oil sample.

In the context of various embodiments, the term "generating" may mean "determining".

The prediction of the at least two constituent oils may be substantially simultaneously generated. No further corroboration of data and no human intervention are required.

The prediction may include at least a type or a percentage amount of each of the at least two constituent oils.

Various embodiments may provide a method for processing lipid contents of at least one oil sample to simulate at least one blend sample and determining compliance of the at least one blend sample with a prescribed requirement. The method may include receiving a first dataset of values associated with lipid contents of the at least one oil sample; simulating the at least one blend sample based on at least part of the first dataset, wherein the at least one simulated blend sample is represented by a second dataset of values associated with lipid contents of the at least one simulated blend sample; and determining whether each value of the second dataset for each simulated blend sample falls within a pre-determined range of a corresponding lipid content in accordance with the prescribed requirement such that if it is determined affirmative, the simulated blend sample complies with the prescribed requirement, and if it is determined negative, the simulated blend sample fails compliance with the prescribed requirement.

In various embodiments, the step of simulating the at least one blend sample may include selecting from the at least part of the first dataset and drawing a mixing proportion from a probability distribution to obtain the at least one simulated blend sample. The probability distribution may be described as above. For example, the step of simulating the at least one blend sample may include selecting from the at least part of the first dataset and drawing a plurality of mixing proportions from the probability distribution to obtain a plurality of simulated blend samples; and wherein each mixing proportion differs from another mixing proportion within a range between a maxima and a minima of the probability distribution.

In various embodiments, the prescribed requirement may include a national standard for oil blends implemented in China. It should be appreciated that other national standards may also be considered.

While each of the methods described above is illustrated and described as a series of steps or events, it will be appreciated that any ordering of such steps or events are not to be interpreted in a limiting sense. For example, some steps may occur in different orders and/or concurrently with other steps or events apart from those illustrated and/or described herein. In addition, not all illustrated steps may be required to implement one or more aspects or embodiments described herein. Also, one or more of the steps depicted herein may be carried out in one or more separate acts and/or phases.

Various embodiments further provide a computer readable storage medium including computer readable instructions operable when executed by a computer to process lipid contents of an oil sample and simulate a training sample, and/or to predict a blending formula of a blended oil sample, and/or to identify a blind oil sample; and/or to process lipid contents of at least one oil sample to simulate at least one blend sample and determine compliance of the at least one blend sample with a prescribed requirement. The computer readable instructions may be configured to perform the method 100 and/or the method 120, in accordance with various embodiments; and/or the method of identifying a blind oil sample; and/or the method of processing lipid contents of at least one oil sample to simulate at least one blend sample and determining compliance of the at least one blend sample with a prescribed requirement, as described hereinabove.

FIG. 1C shows a schematic view of an apparatus or system 140 for processing lipid contents of at least one oil sample and simulating at least one training sample, and/or for predicting a blending formula of a blended oil sample; and/or to identify a blind oil sample; and/or to process lipid contents of at least one oil sample to simulate at least one blend sample and determine compliance of the at least one blend sample with a prescribed requirement, according to various embodiments. In FIG. 1C, the apparatus or system 140 includes a receiving unit 142 configured to receive a dataset of values associated with lipid contents of at least one oil sample, or a blended oil sample, or a blind oil sample; a memory 144 for at least storing a multivariate mixture model capable of accounting for at least a multivariate variance between the lipid contents of the at least one oil sample, a single prediction model capable of generating a prediction of the blending formula of the blended oil sample, and a probability distribution model capable of simulating a blend sample based on at least part of the dataset; and a processor 146 configured to access the multivariate mixture model and the single prediction model stored in the memory 144 to perform steps of the method 100 (FIG. 1A) or steps of the method of identifying the blind oil sample; and steps of the method 120 (FIG. 1B), respectively, for processing the lipid contents of the at least one oil sample and simulating at least one training sample, or for identifying the blind oil sample; and for generating a prediction of the blending formula of the blended oil sample, respectively, in accordance with various embodiments. The processor 146 may further be configured to access the probability distribution model stored in the memory to perform steps of the method of processing lipid contents of the at least one oil sample to simulate at least one blend sample and determining compliance of the at least one blend sample with a prescribed requirement, in accordance with various embodiments. The receiving unit 142, the memory 144 and the processor 146 may be in communication with one another, as depicted by lines 148, 150. The communication may be bi-directional.

The apparatus or system 140 may include the same or like elements or components as those described in the method 100 of FIG. 1A and/or the method 120 of FIG. 1B, and as such, the like elements may be as described in the context of the method 100 of FIG. 1A and/or the method 120 of FIG. 1B, and therefore the corresponding descriptions are omitted here.

In one aspect, various embodiments may be described in a form of an application of a combination of machine learning techniques, namely, (a) Gaussian mixture model to derive the fatty acid parameters of pure constituent oils, (b) Monte Carlo simulation to generate new samples from the parameters learned in (a) above, specifically using mixture proportions drawn from Dirichlet distribution and (c) using deep learning to predict quantitative measures of blend formulations.

Combining multiple machine learning techniques on fatty acid content features of pure oil types (e.g. nine pure oil types) enables quantitative prediction of oil mixtures.

The advantages provided by the methods 100, 120 and the apparatus or system 140 may include as follow:-

- Modeling multivariate Gaussian mixture model to isolate fatty acid parameters relevant to the shape and spread of the multivariate distributions for each oil type. In so doing, the pure oils may be evenly or substantially evenly sampled to be a representative of the population. These parameters may also be used for inference, in other words, predicting the identity of unknown pure oils.
- Simulation of large and complex blend mixing proportions using the Dirichlet distribution, thus ensuring the symmetric nature of the resulting blend mixtures.
- Modeling complex blends using deep learning accounting for the non-linear relationships in the data.

Examples will be described below in forms of experiments conducted to provide a better understanding of the methods 100, 120 and the apparatus or system 140.

*Samples*

A total of 19,765 samples comprising of 9 oil types were profiled for fatty acid content using GC-FID.

*Multivariate Gaussian mixture model*

A multivariate Gaussian mixture model was constructed and constrained by the following geometric characteristics: a diagonal distribution, equal volume and shape.

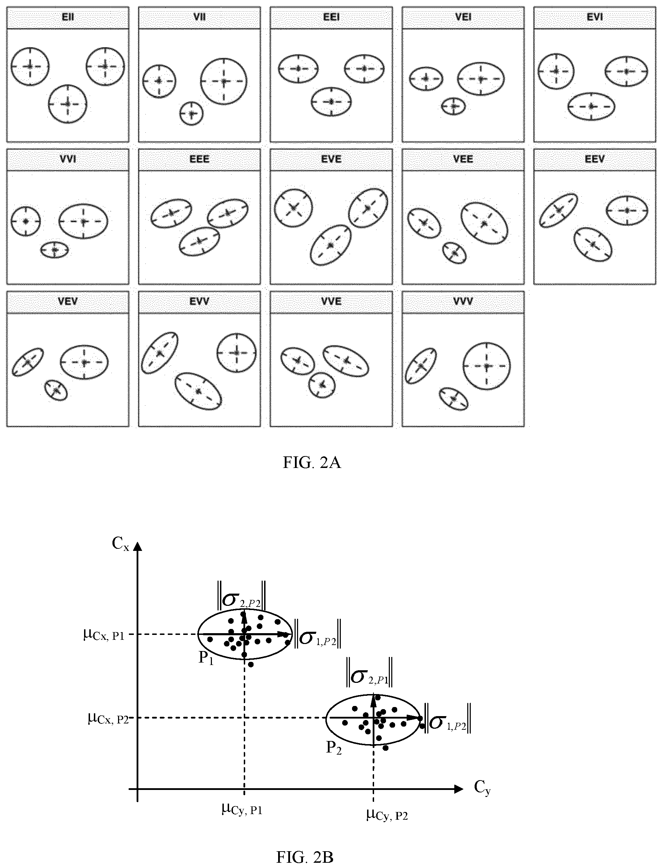

FIG. 2A shows graphical representations illustrating various exemplary configurations of the Gaussian mixture model having constraint on the shape parameters for three clusters. The examples described hereinbelow is based on a similar context of one of the configurations (e.g., the graphical representation of EEI). It should be appreciated that the other configurations may also be applied in constrainting the Gaussian mixture model, in accordance with other embodiments.

Based on the configuration, groups of fatty acid parameters may be determined. Each group of fatty acid parameters may include a vector of average (mean) values representing likely fatty acid percentages and a covariance matrix representing a spread within and across the likely fatty acid percentages. For illustration purposes only to enable a better understanding on the determination of the groups of fatty acid parameters, FIG. 2B shows a geometrical representation of a simplified hypothetical example involving two pure oil samples ($P_1$, $P_2$), each of which having two fatty acid contents as variables (Cx , Cy) and where the configuration of EEI of FIG. 2A is employed. In FIG. 2B, the group of fatty acid parameters for $P_1$ includes the vector ($\mu_{Cx,P1}$, $\mu_{C\gamma,P1}$) and the covariance matrix $\begin{bmatrix} \sigma_{11,P1} & \sigma_{12,P1} \\ \sigma_{21,P1} & \sigma_{22,P1} \end{bmatrix}$ while the group of fatty acid parameters for $P_2$ includes the vector ($\mu_{Cx,P2}$, $\mu_{\varepsilon Cy,P2}$) and the covariance matrix $\begin{bmatrix} \sigma_{11,P2} & \sigma_{12,P2} \\ \sigma_{21,P2} & \sigma_{22,P2} \end{bmatrix}$.

The resulting learned model parameters (in this case, fatty acid parameters) were used to validate the identity of the pure oils, and to simulate new samples to reduce the complexity of the dataset. Twelve (12) groups of oils were identified by the model with each group containing >99% of each of the 9 original oil types. This means some oil types have been broken down into smaller subgroups but the identification accuracy remains as >99%, as shown in Table 1 summarizing the results obtained based on fatty acid contents (FAC).

Table 1

| | Predicted | | | | | | | | | | | |
|---|---|---|---|---|---|---|---|---|---|---|---|---|
| | **1** | **2** | **3** | **4** | **5** | **6** | **7** | **8** | **9** | **10** | **11** | **12** |
| **Real** | | | | | | | | | | | | |
| CO | 0 | 0 | 0 | 0 | 0 | 0 | $1968^h$ | $691^i$ | 0 | 0 | 0 | 0 |
| HERSO | $24^a$ | 0 | $812^d$ | 0 | 0 | $1891^g$ | 0 | 0 | 0 | 0 | 0 | 0 |
| LERSO | $3450^b$ | 0 | 0 | 0 | 0 | 6 | 0 | 0 | 0 | 0 | 0 | 0 |
| LNO | 0 | 0 | 0 | 0 | 0 | 0 | 0 | 0 | $55^j$ | 0 | 0 | 0 |
| PNO | 0 | $1115^c$ | 0 | 0 | 0 | 0 | 0 | 0 | 0 | 0 | 0 | 0 |
| RBO | 0 | 0 | 0 | 0 | 0 | 0 | 0 | 0 | 0 | 0 | 0 | $608^m$ |
| SBO | 0 | 0 | 0 | 0 | $4580^f$ | 0 | 0 | 0 | 0 | $2274^k$ | 0 | 0 |
| SFO | 0 | 0 | 0 | $1318^e$ | 0 | 0 | 0 | 0 | 0 | 0 | 0 | 0 |
| SSO | 0 | 0 | 0 | 0 | 0 | 0 | 0 | 0 | 0 | 0 | $693^l$ | 0 |
| **Identity of cluster** | LERSO | PNO | HERSO | SFO | SBO | HERSO | CO | CO | LNO | SBO | SSO | RBO |
| **Accuracy (%)** | 99.3 | 100 | 100 | 100 | 100 | 99.6 | 100 | 100 | 100 | 100 | 100 | 100 |
| Superscripts a to m in Table 1: corresponds to the points with the respective labels a to m in FIG. 2D. | | | | | | | | | | | | |

The abbreviated oil types indicated in Table 1 are explained in Table 3 below.

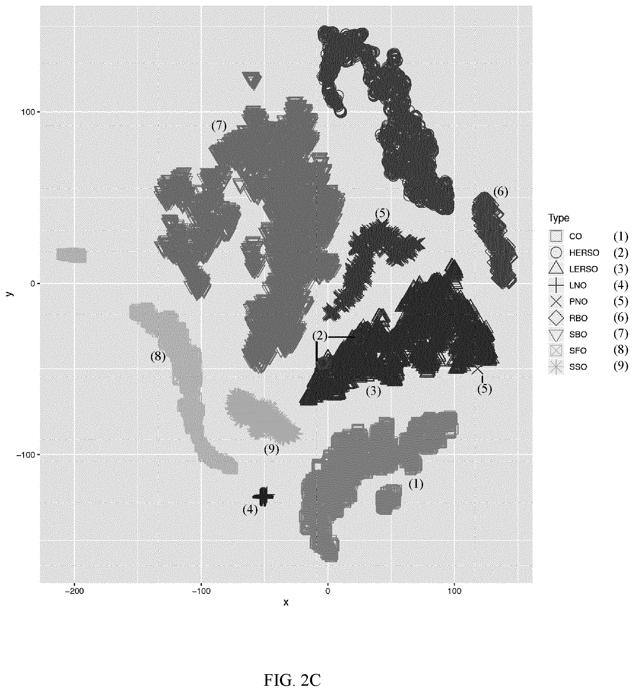

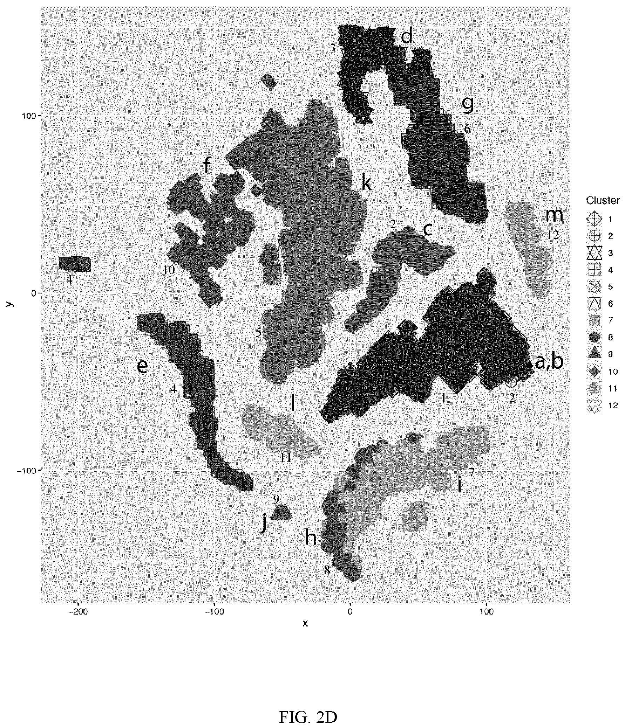

FIG. 2C shows a graphical representation of Table 1 illustrating the clusters of the 9 original oil types in a latent space, where the x and y axes represent arbitary values. FIG. 2D shows a graphical representation of Table 1 illustrating the clusters of the 12 groups of oils identified by the Gaussian mixture model, where the x and y axes represent arbitary values. From these graphical representatios, it is observed that the Gaussian mixture model identifies the pure oils with significantly high levels of accuracy.

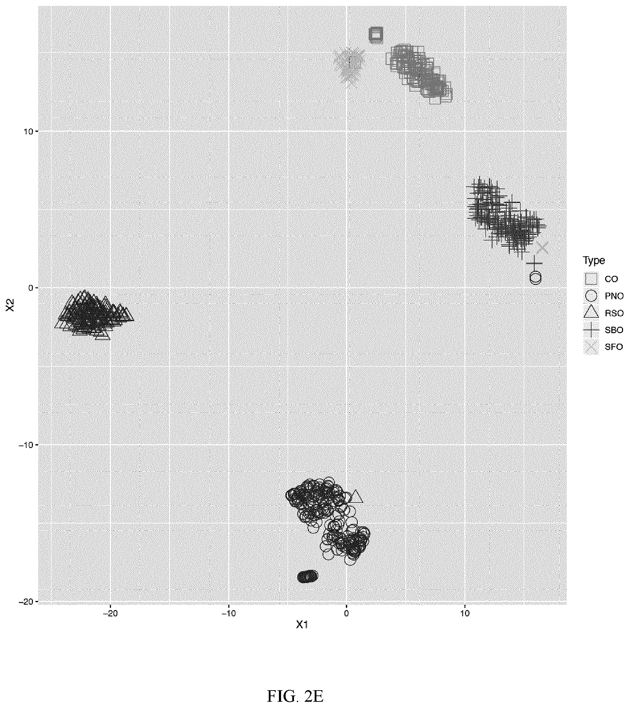

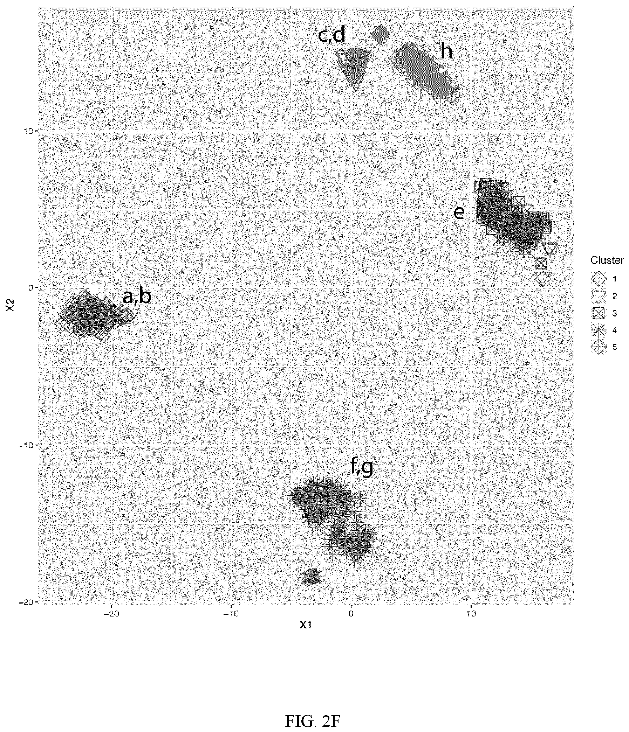

Similar methodology may be applied to TAGs (instead of fatty acid as described above). In this case, the resulting learned model parameters (e.g., TAG parameters) may be used to validate the identity of the pure oils. FIG. 2E shows a graphical representation of an example clustering 5 original oil types based on TAGs, where the $x_1$ and $x_2$ axes represent arbitary values. FIG. 2F shows a graphical representation illustrating the clusters of 5 groups of oil identified by the Gaussian mixture model based on TAGs, where the $x_1$ and $x_2$ axes represent arbitary values. The Gaussian mixture model is able to identify the pure oils based on TAGs. Table 2 summarizes the results obtained based on TAGs.

Table 2

| | Predicted | | | | |
|---|---|---|---|---|---|
| | **1** | **2** | **3** | **4** | **5** |
| **Real** | | | | | |
| CO | 0 | 1[c] | 0 | 0 | 128[h] |
| PNO | 3[a] | 0 | 0 | 208[f] | 0 |
| RSO | 124[b] | 0 | 0 | 1[g] | 0 |
| SBO | 0 | 0 | 156[e] | 0 | 0 |
| SFO | 0 | 67[d] | 0 | 0 | 0 |
| **Identity of cluster** | RSO | SFO | SBO | PNO | CO |
| **Accuracy (%)** | 97.6 | 98.5 | 100 | 99.5 | 100 |
| Superscripts a to h in Table 2: corresponds to the points with the respective labels a to h in FIG. 2F. | | | | | |

Turning back now to FAC, the original and simulated pure oil samples were transformed into a space of reduced dimensions to observe how well the FAC features can separate the pure oils.

Two different spaces, namely, a linear-transformed space and a non-linear transformed space were applied to illustrate the contrasting effect.

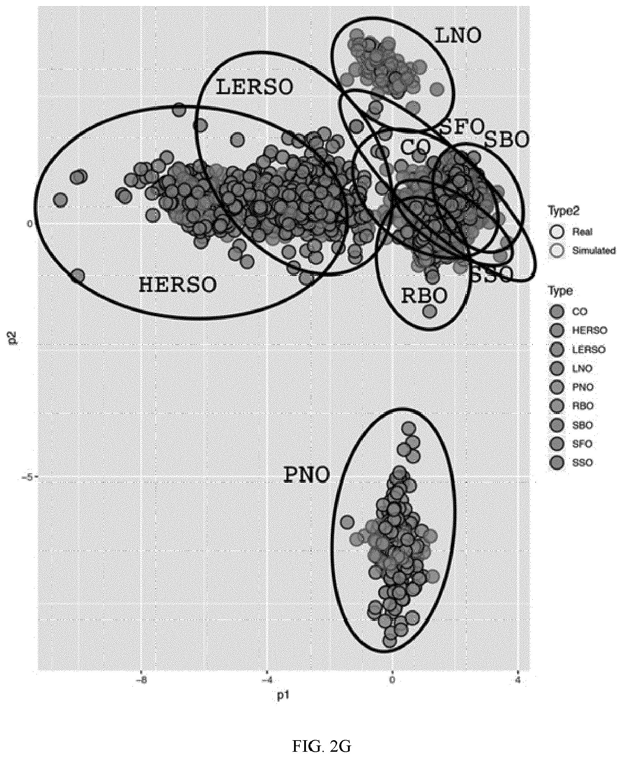

FIG. 2G shows a graphical representation illustrating real and simulated data of pure oils from the Gaussian mixture model in a linear-transformed space (based on FAC). The 9 original oil types, as described in Table 3 below, were commercially purchased from different producers and providers in the Chinese market during the period of January 2016 to December 2017.

Table 3

| Oil type | Oil abbreviation |
|---|---|
| CO | Corn oil |
| SBO | Soybean oil |
| PNO | Peanut oil |
| LNO | Lineseed oil |
| RBO | Rice bran oil |
| LERSO | Low erucic acid rapeseed oil |
| HERSO | High erucic acid rapeseed oil |
| SSO | Sesame oil |
| SFO | Sunflower oil |

In the linear-transformed (PLS) space, FIG. 2G depicts that only four groups out of the 9 oils are separated. Any linear relationship (as noted in existing approaches) may possibly exist among a blend of these four oil groups. The other overlapping samples indicate that linear relationship between other blends of these samples are unlikely.

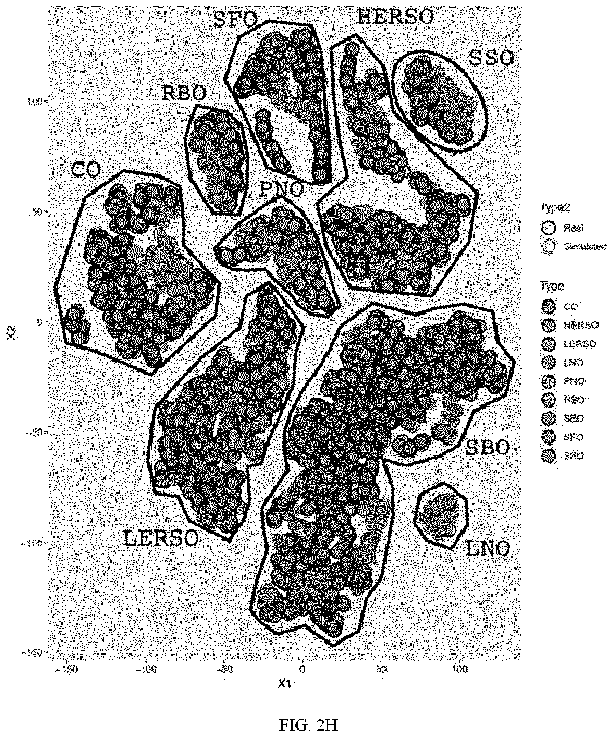

FIG. 2H shows a graphical representation illustrating real and simulated data of pure oils from the Gaussian mixture model in a non-linear transformed space, e.g., in a t-Distributed Stochastic Neighbor Embedding (t-SNE) space. The same 9 original oil types were explored.

In the non-linear transformed (t-SNE) space, FIG. 2H despicts that all oil types are clearly separated. The differences observed between the linear transform (PLS) and the non-linear transform (t-SNE) further supports that for complex blends, a non-linear model may be required.

*Monte Carlo simulation*

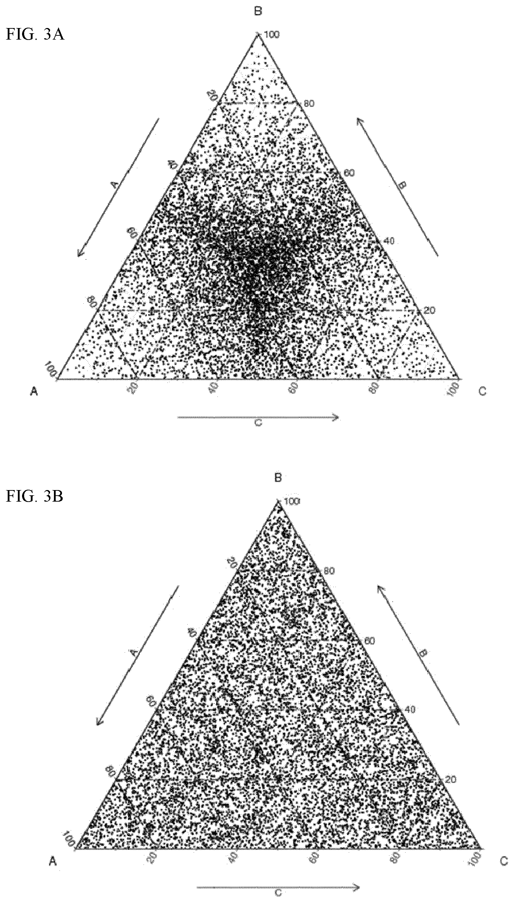

Using the simulated pure oils as mentioned above, additional simulation of blended oil fatty acid content was performed. This was done by firstly, selecting constituent oils out of the 9 pure oils, giving $2^9 - 9 = 493$ combinations, and secondly, drawing random numbers from a 9-dimensional Dirichlet distribution. For each of the 493 combinations and the 9 pure oils (total of 512 options), 100,000 mixing proportions were drawn, and thus, arriving at a total of about 49 million simulated blends. This large number may be required to sufficiently represent the space of blends which is constructed by representative pure oils sampled from the population. The processing of drawing from the Dirichlet distribution is important because of the symmetrical properties, which is otherwise not fulfilled by procedures described in existing approaches. This may be demonstrated by simplified examples as shown in FIGS. 3A and 3B.

FIG. 3A shows a simplified graphical representation using three oil types (A, B, C) for blending and illustrating drawing from a random uniform distribution normalized to unity, as provided by existing approaches. FIG. 3B shows a simplified graphical representation using the same three oil types (A, B, C) for blending and illustrating from the Dirichlet distribution, in accordance with one embodiment. It is observed from FIG. 3A that the distribution points are gathered towards the centroid of the triangular representation. Meanwhile, the distribution points in FIG. 3B are shown to be substantially uniformly spreaded within the triangular representation, thus demonstrating the symmetrical properties observed in the Dirichlet distribution and the symmetry of blend formulas. Every point in the triangular representation in FIG. 3B represents three values that sum to 100% where each value is associated with each oil type (A, B, or C).

The symmetrical properties in the Dirichlet distribution may be more pronounced when a larger number of constituent oils are considered, e.g. 9 oil types.

Although the symmetric nature of the Dirichlet distribution is used for the examples described herein, the asymmetric nature of a Dirichlet distribution may also be used in other embodiments.

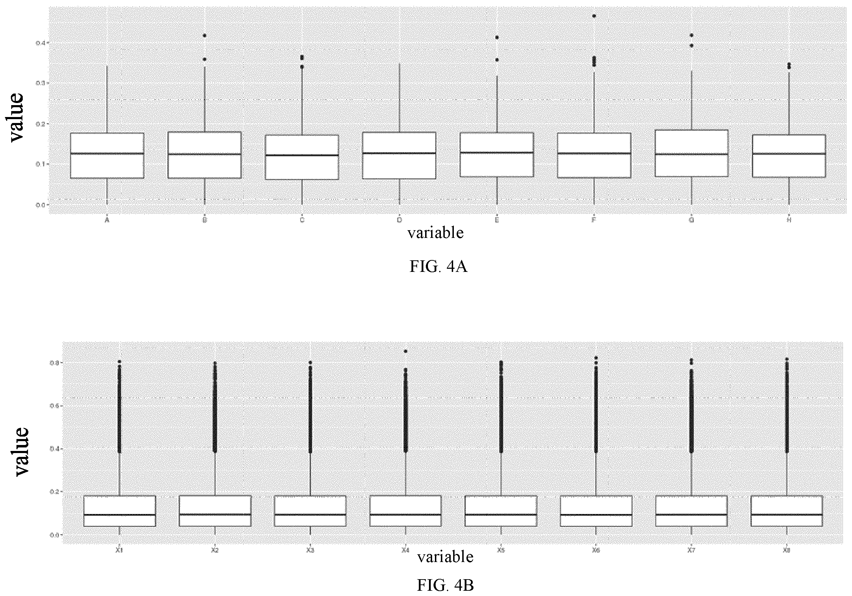

FIG. 4A shows a box plot drawing 9 mixing proportions normalized from a uniform distribution, as provided by existing approaches. FIG. 4B shows a box plot drawing 9 mixing proportions from the Dirichlet distribution, in accordance with one embodiment. It is noted that two of the 9 oils (as indicated in Table 2), more specifically, high erucic acid rapeseed oil and low erucic acid rapeseed oil have been collectively represented as rapeseed oil, thereby resulting 8 discrete variables in the respective box plots.

It is observed from FIG. 4A that although the mean for each variable (oil type) is at a normalized value of about 0.13, the lower quartile and the upper quartile of one variable differ from another variable. The data points outside the whisker of one variable also differs from another variable. Meanwhile, FIG. 4B shows each variable (oil type) having substantially similar mean values, lower quartiles, upper quartiles as well as data points outside the respective whiskers, thereby demonstrating the symmetrical properties in the Dirichlet distribution when using large number of oil types more clearly.

The simulation described above provided the dataset created for modeling which includes the simulated blended oils and the pure oil samples.

*Deep learning model*

Deep learning was used to model the relationship between FAC as generated by the simulation as described above to the mixing proportions (including pure oils). The model may include multiple layers and each layer may be associated with a mathematical manipulation. The models find a correct mathematical manipulation to turn an input (e.g., data containing the fatty acid contents of the simulated oil blends and pure oils) into an output. The model may identify the correct mathematical manipulation by going through the layers with the calculation of the probability of the output.

In a simple form, for mathematical manipulation, each the deep learning model includes a matrix of features ($f_1$ to $f_X$) and each feature is manipulated by a weight. The weight may represent or may be associated with a level of influence on the output. The features may correspond to the values associated with the fatty acid contents of the simulated oil blends and pure oils. It should be appreciated that other features, not mentioned herein, may also be considered.

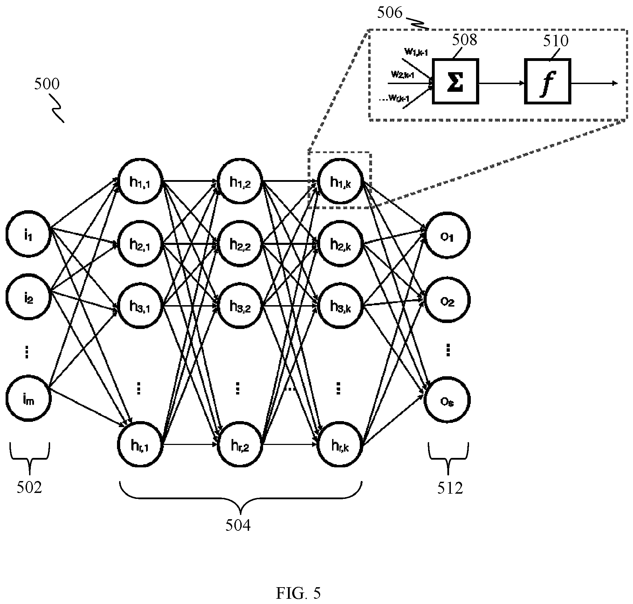

More specifically, the deep learning model, or interchangeably referred to as deep neural network, accepts as input the data. Each of the neural network layer is represented by a number of nodes, initialized with random weights. Data inputs flow through this network by multiplication with the weights associated with a node, through a process of forward propagation. An suitable activation function transforms this result so that non-linearity may be modeled. The activation function may be a sigmoid function or a tanh function. This process may be repeated over the number of layers in the network until finally reaching the output layer where the results are cummulated to represent the quantitative proportions of the oil and its adulterant(s). FIG. 5 shows a schematic representation of the deep learning model 500, in accordance with various embodiments. In FIG. 5, the inputs, $i_m$, 502 include m number of features, e.g., the values associated with the fatty acid contents of the simulated oil blends and pure oils used for modelling. The hidden nodes, $h_{r,k}$, 504 are of size r and k denoting the depth and width of the hidden nodes 504. Each hidden node 504 (as expanded and depicted in a dotted-line area 506) is computing the sum of weights, w, represented by $\Sigma$ 508 and applying an activation function $f$ 510 to estimate non-linear relationships in the data. The outputs, $o_s$, include s number of outputs to estimate the type and quantity of each constituent oil. The initial estimate of the output generally have large errors as the nodes (e.g., 504) of the network 500 was intially assigned random weights. A back propagation algorithm readjusts these weights, w, so that the errors in the outputs 512 may be reduced. This iterative process of forward and backward propagations, changes the weights, w, in the deep neural network 500 until the error is sufficiently small, or if the errors have stopped decreasing. To prevent overfitting of data, a hold-out set of data may usually be reserved for forward propagation to substantially simultaneously check if errors on an independent dataset not trained on the deep neural network 500 also has sufficiently small errors.

*PLS model*

PLS model is a linear model. For example, PLS2 corresponds to the case where there are several dependent variables. This is different from PLS1 which corresponds to the case where there is only one dependent variable. For PLS1, features X are projected into a latent variable space T and one dependent variable y. For PLS2, both the features X and dependent variables Y are matrices, and are both projected into latent variable spaces T and U, where a regression model may be used to ensure that the covariances are maximized.

*Experiments*

Each of the deep learning model and the PLS model were trained by cross-validation and performed by partitioning 90% of the data for training and 10% of the samples for blind testing.

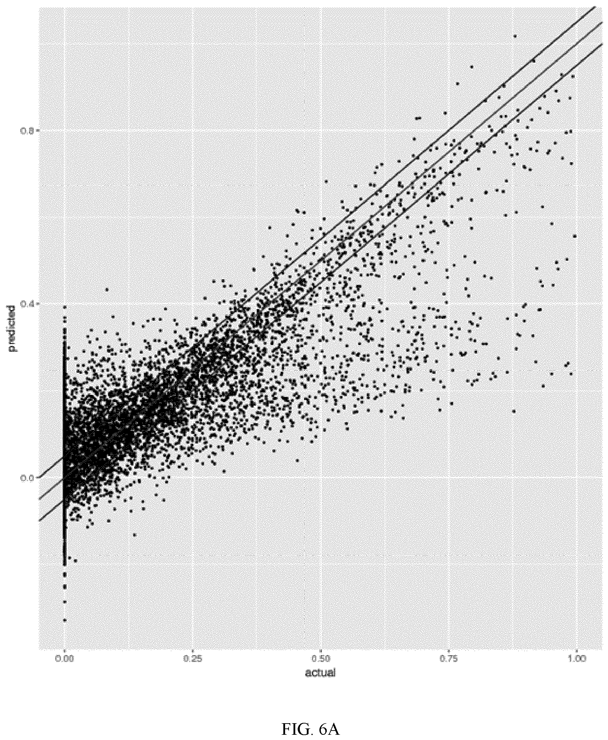

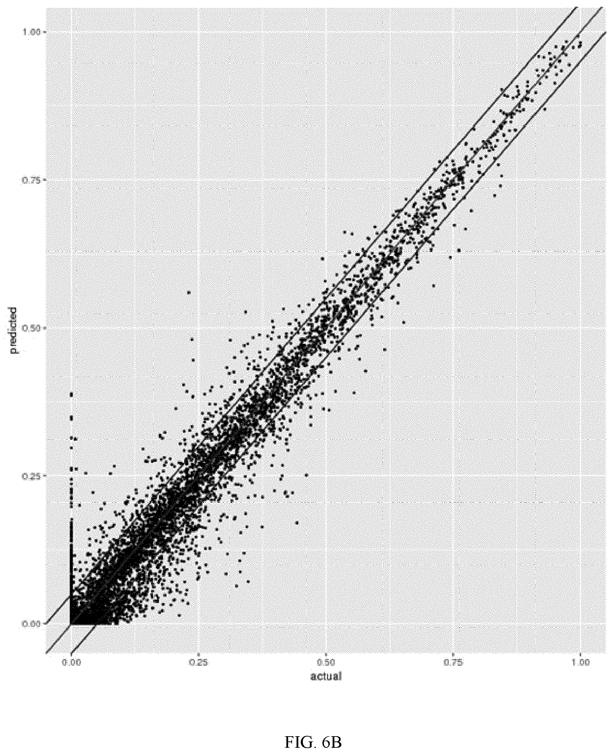

FIG. 6A shows a graph representing the final PLS results for 9 constituent oils. FIG. 6B shows a graph representing the final deep learning results for the same 9 constituent oils.
The final results from the two models were compared against each other to reflect the contrast in accuracy.
From the final PLS results, it can be observed that the prediction of some samples may not reflect the actual percentages correctly. This may cause some uncertainty in the prediction of the type and amount of constituent oils in the blended oil sample. In other words, large number of blended examples may make methods that depend on linear matrix decomposition described in existing approaches inefficient. However, the PLS model may be applicable for blended oil samples where the number of constituent oils are relatively low (e.g., <3) and where the distinction of different constituent oils are not crucial.
In contrast, FIG. 6B shows substantially consistent predicted values close to the actual values through the entire normalized range using the deep learning model.
In accordance with the final results, the deep learning model is able to quantitatively predict the blended mix to a much higher accuracy with a root-mean-square error (RMSE) of 0.02 compared to a RMSE of 0.11 for PLS.
In another aspect, various embodiments may provide a method involving simulated blended oil lipid contents for checking national standards. For example, the national standards in China for blended oil require lipid contents to fulfil certain ranges, and there are no known no computational method to check against the standards. It is possible to theorize the lipid contents of a blended oil from constituent oil by computing a linear combination of the lipid contents of the constituent oils, for example, C14_blend = ratiol * C14_constituent1 + ratio2 * C14_constituent2. The same concept may be applied to theorize other lipid contents such as saturated fatty acids (*SFA*), unsaturated fatty acids (*USFA*), poly unsaturated fatty acids (*PUFA*), omega3s (n3) and omega6s (n6). In other words, this sum (linear combination) may be used as a checking step. However, theorized blended oil lipid contents rarely meet this equality in the above mentioned linear combination.
The probablity distribution may the Dirichlet distribution and may be used to simulate possible mixing proportions and compute the corresponding blended oil lipid contents. As the national standards (*NS*) are to ensure that *SFA, USFA, PUFA,* n3 and n6 are within a certain range, simulated lipid contents may *be* checked to find mixing ratios that are close to the theorized mixing proportions. In cases where no possible mixing proportions are within specified ranges, the computational method may then determine that the theorized mixing proportion should not be used for blending.

For illustrative purposes, an example using three lipid contents (*SFA, USFA, PUFA*) for two constituent oils denoted by *P*1 and *P*2 will be described here to enable better undertsanding of this aspect.

The input (first) dataset may be represented by:

$$\begin{bmatrix} P1 \\ P2 \end{bmatrix} = \begin{bmatrix} SFA\_P1 & USFA\_P1 & PUFA\_P1 \\ SFA\_P2 & USFA\_P2 & PUFA\_P2 \end{bmatrix}.$$

Assuming the two possible mixing proportions, as denoted by (*M*1, *M*2) and *(M3, M4),* then the two following simulated blend may be:

$$Blend1 = M1P1 + M2P2 = M1\begin{bmatrix} SFA\_P1 \\ USFA\_P1 \\ PUFA\_P1 \end{bmatrix} + M2\begin{bmatrix} SFA\_P2 \\ USFA\_P2 \\ PUFA\_P2 \end{bmatrix};$$

and

$$Blend2 = M3P1 + M4P2 = M3\begin{bmatrix} SFA\_P1 \\ USFA\_P1 \\ PUFA\_P1 \end{bmatrix} + M4\begin{bmatrix} SFA\_P2 \\ USFA\_P2 \\ PUFA\_P2 \end{bmatrix}.$$

(*M*1, *M*2) may be used for blending if

$$M1\begin{bmatrix} SFA\_P1 \\ USFA\_P1 \\ PUFA\_P1 \end{bmatrix} + M2\begin{bmatrix} SFA\_P2 \\ USFA\_P2 \\ PUFA\_P2 \end{bmatrix} = \begin{bmatrix} SFA\_NS \pm Range\_SFA \\ USFA\_NS \pm Range\_USFA \\ PSFA\_NS \pm Range\_PSFA \end{bmatrix}$$

where *SFA_NS ± Range SFA* is a specific range for *SFA, USFA_NS ± Range_USFA* is a specific range for *USFA,* and *PSFA_NS ± Range_PSFA* is a specific range for *PSFA* in accordance with the national standards.

Otherwise, (*M*1, *M*2) should not be used for blending.

Similarly, *(M3, M4)* may be used for blending if

$$M3\begin{bmatrix} SFA\_P1 \\ USFA\_P1 \\ PUFA\_P1 \end{bmatrix} + M4\begin{bmatrix} SFA\_P2 \\ USFA\_P2 \\ PUFA\_P2 \end{bmatrix} = \begin{bmatrix} SFA\_NS \pm Range\_SFA \\ USFA\_NS \pm Range\_USFA \\ PSFA\_NS \pm Range\_PSFA \end{bmatrix}.$$

Otherwise, *(M3, M4)* should not be used for blending.

Although the selection from the Dirchlet distribution and the application of the deep learning model are explained above in the context of FAC, it should be appreciated and understood that similar selection steps and the deep learning model may be applied to TAG to identify the groups of oils.

While the invention has been particularly shown and described with reference to specific embodiments, it should be understood by those skilled in the art that various changes in form and detail may be made therein without departing from the spirit and scope of the invention as defined by the appended claims. The scope of the invention is thus indicated by the appended claims and all changes which come within the meaning and range of equivalency of the claims are therefore intended to be embraced.

**Claims**

1. A method for processing lipid contents of at least one oil sample and simulating at least one training sample, the method comprising:

   receiving a first dataset of values associated with lipid contents of the at least one oil sample, wherein the lipid contents of the at least one oil sample comprises a first lipid content and a second lipid content;
   applying a multivariate mixture model to the first dataset of values to simulate an intermediate dataset, wherein the multivariate mixture model is capable of accounting for at least a multivariate variance between the value associated with the first lipid content and the value associated with the second lipid content; and
   simulating the at least one training sample based on at least part of the simulated intermediate dataset, wherein the at least one simulated training sample is represented by a second dataset of values associated with lipid contents of the at least one simulated training sample.

2. The method of Claim 1, wherein the multivariate mixture model comprises a Gaussian mixture model or a multivariate Gaussian mixture model, preferably the multivariate mixture model is constructed and constrained by a geometric characteristic, wherein preferably the geometric characteristic comprises a diagonal distribution with equal volume and equal shape.

3. The method of Claim 1 or 2, wherein the step of applying the multivariate mixture model to the first dataset of values comprises:

   learning groups of lipid parameters from the first dataset of values; and
   simulating, based on the groups of lipid parameters, the intermediate dataset;

   wherein preferably each group of lipid parameters comprises a vector of average values representing likely lipid percentages and a covariance matrix representing a spread within and across the likely lipid percentages.

4. The method of any one of claims 1 to 3, wherein the at least one oil sample comprises a pure oil sample.

5. The method of any one of claims 1 to 4, wherein the step of simulating the at least one training sample comprises performing a Monte Carlo simulation; preferably the step of simulating the at least one training sample comprises: selecting from the at least part of the simulated intermediate dataset and drawing a mixing proportion from a probability distribution to obtain the at least one simulated training sample; wherein the probability distribution preferably comprises a Dirichlet distribution, wherein the number of dimensions of the Dirichlet distribution is preferably more than or equal to 2, more preferably 9 or more.

6. The method of any one of claims 1 to 5, wherein the lipid contents of the oil sample comprises fatty acid contents of the oil sample obtainable using a gas chromatography - flame ionization detector or photonic detection or comprises triacylglyceride (TAG) contents.

7. A method for predicting a blending formula of a blended oil sample, the method comprising:

   receiving a dataset of values associated with lipid contents of the blended oil sample;
   providing a single prediction model capable of generating a prediction of at least two constituent oils in the blended oil sample; and
   processing the at least part of the dataset using the single prediction model,

   wherein the single prediction model is trained by a training dataset, wherein the training dataset comprises a second dataset of values associated with lipid contents of the at least one training sample simulated by a method of any one of claims 1 to 6, preferably the single prediction model comprises a deep neural network.

8. The method of Claim 7, further comprising generating the prediction of at least two constituent oils in the blended oil sample, wherein preferably the prediction comprises at least a type or a percentage amount of each of the at least two constituent oils.

9. A method for identifying a blind oil sample, the method comprising:

receiving a dataset of values associated with lipid contents of the blind oil sample; and

applying a multivariate mixture model comprising groups of lipid parameters to the dataset of values to identify the blind oil sample,

wherein the groups of lipid parameters is learned by the multivariate mixture model based on lipid contents of at least one preceding oil sample, wherein the lipid contents of the at least one preceding oil sample comprise a first lipid content and a second lipid content, and wherein the multivariate mixture model is capable of accounting for at least a multivariate variance between a value associated with the first lipid content and a value associated with the second lipid content.

10. A method for processing lipid contents of at least one oil sample to simulate at least one blend sample and determining compliance of the at least one blend sample with a prescribed requirement, the method comprising:

receiving a first dataset of values associated with lipid contents of the at least one oil sample;

simulating the at least one blend sample based on at least part of the first dataset, wherein the at least one simulated blend sample is represented by a second dataset of values associated with lipid contents of the at least one simulated blend sample; and

determining whether each value of the second dataset for each simulated blend sample falls within a predetermined range of a corresponding lipid content in accordance with the prescribed requirement such that if it is determined affirmative, the simulated blend sample complies with the prescribed requirement, and if it is determined negative, the simulated blend sample fails compliance with the prescribed requirement.

11. The method of Claim 10, wherein the step of simulating the at least one blend sample comprises selecting from the at least part of the first dataset, and

drawing a mixing proportion from a probability distribution to obtain the at least one simulated blend sample, preferably drawing a plurality of mixing proportions from the probability distribution to obtain a plurality of simulated blend samples; and wherein each mixing proportion differs from another mixing proportion within a range between a maxima and a minima of the probability distribution.

12. The method of Claim 10 or 11, wherein the prescribed requirement comprises a national standard for oil blends implemented in China.

13. A computer readable storage medium comprising computer readable instructions operable when executed by a computer to process lipid contents of at least one oil sample and simulate at least one training sample, the computer readable instructions configured to perform a method of any one of claims 1 to 14; and/or to predict a blending formula of a blended oil sample, the computer readable instructions configured to perform a method of any one of claims 15 to 18; and /or to identify a blind oil sample, the computer readable instructions configured to perform a method of claim 19; and/or to process lipid contents of at least one oil sample to simulate at least one blend sample and determine compliance of the at least one blend sample with a prescribed requirement, the computer readable instructions configured to perform a method of any one of claims 10 to 12.

14. An apparatus or system comprising:

a receiving unit configured to receive a dataset of values associated with lipid contents of at least one oil sample, or a blended oil sample, or a blind oil sample;

a memory for at least storing a multivariate mixture model capable of accounting for at least a multivariate variance between the lipid contents of the at least one oil sample, a single prediction model capable of generating a prediction of a blending formula of the blended oil sample; a probability distribution model capable of simulating a blend sample based on at least part of the dataset; and

a processor configured to access the multivariate mixture model stored in the memory to perform steps of a method of any one of claims 1 to 6 for processing the lipid contents of the at least one oil sample and simulating at least one training sample, or to perform steps of a method of claim 9 for identifying the blind oil sample; the single prediction model stored in the memory to perform steps of a method of claim 7 or 8 for generating a prediction of the blending formula of the blended oil sample; and the probability distribution model stored in the memory to perform steps of a method of any one of claims 10 to 12 for processing lipid contents of the at least one oil sample to simulate at least one blend sample and determining compliance of the at least one blend sample with a prescribed requirement.

100

| Receive a first dataset of values associated with lipid contents of at least one oil sample | 102 |

| Applying a multivariate mixture model to the first dataset of values to simulate an intermediate dataset | 104 |

| Simulate at least one training sample based on at least part of the simulated intermediate dataset | 106 |

FIG. 1A

120

| Receive a dataset of values associated with lipid contents of a blended oil sample | 122 |

| Provide a single prediction model capable of generating a prediction of at least two constituent oils in the blended oil sample | 124 |

| Process the at least part of the dataset using the single prediction model | 126 |

FIG. 1B

140

| Apparatus or system | |
| 142 — Receiving unit | |
| | — 148 |
| 146 — Processor | |
| | — 150 |
| 144 — Memory | |

FIG. 1C

FIG. 2A

FIG. 2B

FIG. 2C

FIG. 2D

FIG. 2E

FIG. 2F

FIG. 2G

FIG. 2H

FIG. 3A

FIG. 3B

FIG. 4A

FIG. 4B

FIG. 5

FIG. 6A

FIG. 6B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SCURUCCA et al.** mclust 5 : Clustering, Classification and Density Estimation Using Gaussian Finite Mixture Models. *The R Journal,* August 2016, vol. 8 (1